# EUROPEAN PATENT APPLICATION

(11) **EP 3 653 720 A1**
(43) Date of publication of application: **20.05.2020**
(21) Application number: 18206737.1
(22) Date of filing: 16.11.2018
(51) Int. Cl.: C12P 5/02

(54) **A METHOD FOR PROCESSING BIOMASS DIGESTATE**

(71) Applicant: NGF Nature Energy Biogas A/S, 5220 Odense SØ (DK)
(72) Inventor: JEPPESEN, Martin Dan, 5200 Odense V (DK); GYLLENBORG, Morten Enzo, 5700 Svendborg (DK); NIELSEN, Sine Stylsvig, 5000 Odense C (DK)
(74) Representative: Patentgruppen A/S

(57) **Abstract**

A method for processing biomass digestate is disclosed. The method comprises the steps of
- providing a biomass digestate (BD),
- subjecting the biomass digestate to a separation step (SEP) into a liquid fraction (LF) and a solid biomass digestate fraction (SBD),
- subjecting the solid biomass digestate fraction (SBD) to an acidic wash step (AW) at a pressure below 2 bar to obtain a washed biomass digestate (WBD),
- separating (SEP) the washed biomass digestate (WBD) into a liquid wash fraction (LW) and a solid wash fraction (SW),
- posttreating (POST) the solid wash fraction (SW) to obtain a posttreated solid wash fraction (PSW) and
- anaerobic digesting (AD) the posttreated solid wash fraction (PSW) to obtain a posttreated biomass digestate (PBD)
wherein the amount of phosphorous in the solid wash fraction (SW) is reduced compared to amount of phosphorous in the solid biomass digestate fraction (SBD).

## Description

### FIELD OF INVENTION

The invention relates to a process for processing biomass digestate for obtaining e.g. biogas, lignin and nutrients like phosphorus and nitrogen.

### BACKGROUND

Biomass digestate is a product of digestion of biomass and represents the digested material, which is removed from the digestor after recovery of biogas.

Digestate has most often a rich content of nutrients including nitrogen (N), phosphorus (P), potassium (K) and sulfur (S). Digestate may thus be applied as fertilizer to crops without further processing, however this has the disadvantage that the concentration of e.g. phosphorus and nitrogen and especially their relative amount i.e. ratio may not be controlled or adjusted according to e.g. regulations or crop requirements. Furthermore, deposition of biomass digestate is costly. In addition, many valuable products present in the biomass digestate, such as organic matter that may be converted into biogas or purified lignin, is not utilized.

It is an object of the present invention to solve the above problems.

### SUMMARY

The invention relates to a method for processing biomass digestate, the method comprising the steps of
- providing a biomass digestate BD,
- subjecting the biomass digestate to a separation step SEP into a liquid fraction LF and a solid biomass digestate fraction SBD,
- subjecting the solid biomass digestate fraction SBD to an acidic wash step AW at a pressure below 2 bar to obtain a washed biomass digestate WBD,
- separating SEP the washed biomass digestate WBD into a liquid wash fraction LW and a solid wash fraction SW,
- posttreating POST the solid wash fraction SW to obtain a posttreated solid wash fraction PSW and
- anaerobic digesting AD the posttreated solid wash fraction PSW to obtain a posttreated biomass digestate PBD
wherein the amount of phosphorous in the solid wash fraction SW is reduced compared to amount of phosphorous in the solid biomass digestate fraction SBD.

The present invention advantageously utilizes biomass digestate to obtain valuable products such as nutrients for agricultural use and high value products such as biogas and lignin.

A significant advantage of the invention is that a biomass digestate can be utilized to provide valuable products at a relatively low cost. This may reduce cost in biogas production, since a lesser amount of digestate is produced and a lesser amount of waste thus has to be disposed of, which is costly.

Another advantage of the invention is that nutrients, such as phosphorus and nitrogen, can be recovered individually, and that the individual concentrations of nutrients and especially their relative amount i.e. ratio may thus be controlled and adjusted according to e.g. regulations or crop requirements, when used as fertilizer. The liquid wash fraction, for example, comprises a high amount of the nutrient phosphorus originally contained in the biomass digestate and may thus be utilized as e.g. a biofertilizer in desired amounts.

A further advantage is that the digestibility of the biomass digestate may be improved resulting in quicker digestion.

The present invention also provides for significantly increasing the total amount of biogas obtainable from a certain amount of biomass. The methane production may according to the method of the invention in this way be increased without adding more biomass, as the biomass is better utilized. It may be increased with more than 10% or more than 20% or even more than 30%.

A further advantage of the invention is that the posttreated biomass digestate may comprise lignin of a high purity and quality, since lignin may in this way be produced with a substantially reduced amount of nutrients, such as P and N, and carbohydrates or even substantially free from nutrients and carbohydrates. Thus, providing a cleaner and higher value lignin. The lignin may be used e.g. as bio-pellets or as replacement for bitumen and other high value green chemicals.

It has thus surprisingly been discovered by the inventors that by employing first an acidic wash step for removing and recovering phosphorus, and then posttreating the biomass digestate, high amounts of both phosphorus, biogas, nitrogen and lignin may be obtained in high purity.

According to an advantageous embodiment of the invention, the acidic wash step is non-pressurized.

All of the above advantages are also obtained when the acidic wash step is non-pressurized, and in addition the method and especially the acidic wash step may be performed easier (for example in a continuous way) and cheaper, when the acidic wash step is non-pressurized, since it may for be performed in an open system or may be performed in reactors with over-/underpressure valves. When the acidic wash step is non-pressurized, the severity of the acidic wash is also lower than when a higher pressure is applied, and thus milder conditions are obtained.

According to an embodiment of the invention, the amount of phosphorous in the solid wash fraction is less than 10% by weight of the phosphorous in the biomass digestate. By subjecting the solid fraction of biomass digestate to an acidic wash step a large proportion of phosphorus can be removed, such as more than 90%, or even more than 95 or 98%, or even substantially all the phosphorus that was present in the biomass digestate may be removed.

According to an embodiment of the invention, the pH in the acidic wash step is lower than 6, such as between 0 to 6, or lower than 4, such as between 0 to 4.

It has been shown by the present inventors that more phosphorus can be removed by lowering pH.

According to an embodiment of the invention, the temperature in the acidic wash step is between 50 to 100 degrees Celsius.

The acidic wash may be more effective at removing (reducing) phosphorus at a temperature of around 90 to 100 degrees Celsius or close thereto, however from an economical view point a lower temperature such as around 50 to 70 degrees Celsius may be more favorable. When the acidic wash step is performed at temperatures not higher than 100 degrees Celsius, it has the advantage to be able to be performed in an open continuous system. Temperatures above 100 degrees Celsius have the potential draw-back than a closed container with pressure is required, which means that it is not possible to run it in a continuous way. At temperatures above 100 degrees Celsius, compounds that inhibit the subsequent anaerobic digestion may also be formed.

According to an embodiment of the invention, the acidic wash step is performed for between 10 minutes to 24 hours, such as between 10 minutes to 10 hours, such as between 10 minutes and 5 hours.

The acidic wash step can be effective in removing phosphorus by being performed in only a short time, such as e.g. 10 minutes, however increasing the incubation time i.e. the time the acidic wash step is performed can increase the amount of phosphorus removed from the solid biomass digestate fraction. If the acidic wash step is performed for a short time, the amount of phosphorus removed from the solid biomass digestate fraction may also be increased by raising the concentration of the acid or increasing the temperature.

According to an embodiment of the invention, the concentration of the acid in the acidic wash step is between 0.01 to 1 mol/liter, such as between 0.04 to 0.5 mol/liter, such as between 0.04 to 0.2 mol/liter.

By increasing the concentration of acid more phosphorus may be removed.

According to an embodiment of the invention, the acid in the acidic wash step is a strong acid.

The strong acid may e.g. be sulphuric acid, hydrochloric acid, or oxalic acid or any combination thereof.

According to an embodiment of the invention, the acid in the acidic wash step is a weak acid.

The weak acid may be a weak organic acid like e.g. acetic acid, citric acid, formic acid or lactic acid.

According to an embodiment of the invention, the amount of phosphorus in the liquid wash fraction is reduced to obtain a low phosphorus liquid fraction.

The amount of phosphorus in the liquid wash fraction may be may be reduced ie. removed by more than 80% by weight, such as more than 90% by weight, such as more than 95% by weight or even substantially all the phosphorus may be removed from the liquid wash fraction.

According to an embodiment of the invention, the amount of phosphorus in the liquid wash fraction LW is reduced by subjecting the liquid wash fraction LW to ion exchange IEX.

By using ion exchange a selective removal of phosphorus, by e.g. not removing nitrogen, as well as very high amount of phosphorus may be removed, such as more than 95% and even more than 98% or even almost all the phosphorus present in the liquid wash fraction may be removed by ion exchange. Thus, ion exchange represents a very efficient and selective way of removing phosphorus from the liquid wash fraction and thus a corresponding high amount of phosphorus may be precipitated as e.g. Na3PO4 by subjecting the ion exchange resin to NaOH. The phosphorus that is removed from the liquid wash fraction may then be used as e.g. a fertilizer and has the advantage that it may be substantially free from other nutrients such as nitrogen.

The amount of phosphorus in the liquid wash fraction may also be reduced by e.g. precipitation with Ca(OH)2 or as struvite or by reverse osmosis.

According to an embodiment of the invention, the ion exchange is performed by passing the liquid wash fraction over a resin.

The ion exchangers may e.g. be a strongly basic anion exchanger, such as Amberjet 4200 from Merck or an ion exchanger comprising a polysterene-based resin, such as Lewatit FO 36 from Lanxess or a mixed bed ion exchanger such as Amberlite MB-3 from Merck.

According to an embodiment of the invention, the liquid wash fraction LW is recirculated into the acidic wash step AW.

By recycling the low phosphorus wash fraction into the acidic wash step, the acid which is present in the low phosphorus wash fraction may advantageously be reused and thus reducing cost as the method requires less use of acid and water.

According to an embodiment of the invention, the biomass digestate BD is provided by heating a soft biomass SB in a pretreatment step PRE followed by anaerobic digesting AD of the soft biomass SB to obtain the biomass digestate BD.

An advantage of the pretreatment step is that it makes the soft biomass more accessible for subsequent degradation for example by melting pectin and other waxes away so that e.g. hemicellulose is more accessible for digestion. It may thus also make the resulting biomass digestate more accessible for recovery of nutrients.

According to an advantageous embodiment of the invention, the pretreatment is non-pressurized.

According to an embodiment of the invention, the pretreatment temperature is between 65 to 100 degrees Celsius, such as between 65 to 90 degrees Celsius, such as between 65 to 80 degrees Celsius or such as between 70 to 90 degrees Celsius.

An advantage of the above embodiment is that relatively low temperatures below 100 degrees Celsius in the pretreatment step may allow in a synergistic way the temperature in the posttreatment step to be higher than in conventional methods since less inhibitory compounds, such as furfural, 5-HMF and acetic acid, will be formed. This is especially the case when the pretreatment step is non-pressurized. As a result, e.g. more lignin may be melted in the posttreatment step, since higher temperatures of e.g. more than 180 degrees Celsius can be employed, allowing for a more efficient degradation of cellulose especially in the posttreatment step and for obtaining higher yields of biomass degradation products, such as biogas and also lignin.

A non-pressurized pretreatment step involving heating the soft biomass to at a pretreatment temperature between 65 to 100 degrees Celsius and posttreating the biomass in a pressurized posttreatment step by heating the solid wash fraction to a posttreatment temperature above 150 degrees Celsius may give rise to a higher yield of biogas. This may be due to a higher liberation of hemicellulose and cellulose accessible for degradation, without the drawback that also many process inhibitors are formed during the process conditions.

A non-pressurized pretreatment step involving heating the soft biomass to at a pretreatment temperature between 65 to 100 degrees Celsius may also result in a higher effect of the posttreatment, when posttreating the biomass involves laccase treatment, since pretreatment e.g. provides for a faster and/or increased degradation of the soft biomass so that less carbohydrates are surrounding the lignin, which makes the lignin more accessible to the laccases.

According to an embodiment of the invention, the pretreatment step is performed for 2 hours or less.

According to an embodiment of the invention, the pH in the pretreatment step is between 2 to 9, such as between 3 to 7, such as between 3 to 4.

According to an embodiment of the invention, acid and/or food waste is added to the soft biomass.

According to an embodiment of the invention, the anaerobic digesting of the pretreated biomass is performed for less than 10 days.

According to an embodiment of the invention, the amount of hemicellulose in the biomass digestate after the first anaerobic digesting step is less than 40% by weight of the amount of hemicellulose in the soft biomass prior to the first anaerobic digesting step, such as less than 30% by weight.

Thus, a reduction of the amount of hemicellulose of at least 60% by weight of the initial content is obtained.

According to an embodiment of the invention, the posttreatment step is pressurized. This may for example be the case with steam explosion.

According to an embodiment of the invention, the posttreatment temperature is between 150 to 230 degrees Celsius, such as between 170 to 210 degrees Celsius, such as between 180 to 200 degrees Celsius.

According to an embodiment of the invention, the posttreatment step is performed for less than 1 hour.

According to an embodiment of the invention, the posttreatment is performed by steam explosion and/or laccase treatment.

Laccases (EC.1.10.3.2) are copper-containing oxidase enzymes found in many plants, fungi, and microorganisms. Laccases act on phenols and similar molecules, performing one-electron oxidations, which remain poorly defined.

Laccase treatment may advantageously be performed subsequent to steam explosion. Thereby may a larger amount of biogas be obtained, when the posttreatment is followed an anaerobic digestion. Laccase treatment may be performed e.g. at a temperature around 50 degrees Celsius, such as from 40 to 60 degrees Celsius. When the temperature is too high the activity of laccase is destroyed.

According to an embodiment of the invention, the posttreatment is performed by bacteria or fungi treatment.

According to an embodiment of the invention, furfural and 5-HMF and 2-furioc acid are generated in a combined amount of less than 5% by weight in the post treatment step.

According to an embodiment of the invention, anaerobic digesting of the posttreated solid wash fraction is performed for less than 30 days.

The anaerobic digesting step may be performed by bacteria, such as fermentative bacteria, such as ruminococcus albus, ruminococcus flavefaciens and/or fibrobacter succinogenes.

According to an embodiment of the invention, the method further comprises a further separation step SEP after anaerobic digesting AD the posttreated solid wash fraction PSW to obtain a liquid posttreated biomass digestate fraction LPBD and a solid posttreated biomass digestate fraction SPBD.

Separation may for example be performed with a screw press, filter press or decanter.

According to an embodiment of the invention, the method further comprises a drying step DRY of the solid posttreated biomass digestate fraction SPBD to obtain a dried posttreated biomass digestate DPBD.

By drying the solid posttreated biomass digestate fraction, ammonia may advantageously be removed by evaporation and condensed. The ammonia may then for example be bobbled through sulfuric acid to generate ammonia sulphate, which can be used as e.g. fertilizer. Furthermore, dry lignin may thus be obtained in a purity of more than 40% by weight, since other components such as phosphorus and nitrogen are removed.

According to an embodiment of the invention, the amount of nitrogen in the dried posttreated biomass digestate is reduced by at least 50%, such as at least 60%, such as at least 70% or such as at least 80% by weight of the nitrogen comprised in the biomass digestate entering the process.

In other words, the amount of nitrogen in the dried posttreated biomass digestate comprises less than 50% of the nitrogen comprised in the biomass digestate or even less than 20%.

According to an embodiment of the invention, at least 50%, such as at least 60%, such as at least 70% or such as at least 80% by weight of the nitrogen comprised in the biomass digestate entering the process may be isolated.

According to an embodiment of the invention, lignin is obtained from the method in a purity of at least 30% by weight, such as at least 35% by weight, such as at least 40% by weight.

By subjecting the biomass digestate to the wash step prior to posttreatment and then drying, the purity of lignin may be substantially increased to more than 30% or more than 35% or more than 40% or even higher, since e.g. carbohydrates and nutrients, such as phosphorus and nitrogen are removed, which increases the value of lignin. Lignin may also be obtained from the posttreated biomass digestate by e.g. extraction with an organic solvent such as ethanol or methanol.

According to an embodiment of the invention, lignin LIG is comprised in the posttreated biomass digestate PBD or dried posttreated biomass digestate DPBD in an amount of more than 30% by weight, such as in an amount of more than 35% by weight, such as in an amount of more than 40% by weight.

Lignin may be produced substantially free from nutrients and/or carbohydrates, thus obtaining lignin of high quality, which may be used for e.g. bio-pellets or replacement for bitumen and other high value green chemicals.

According to an embodiment of the invention, biogas BG is obtained from at least the anaerobic digesting step AD of the posttreated solid wash fraction PSW.

According to an embodiment of the invention, the method further comprises recirculation of at least a part of a liquid fraction from any separation step (SEP) to any anaerobic digesting step (AD) and/or the pretreatment step.

In this way liquid and bacteria are advantageously reused, thus reducing the cost of the method.

According to an embodiment of the invention, a system is arranged to operate according to the method of the invention or any of its embodiments.

### FIGURES

Figure 1 illustrates a method for processing biomass digestate according to an embodiment of the invention.
Figure 2 illustrates a method for processing biomass according to an embodiment of the invention.
Figure 3 illustrates removal of phosphorus from a solid biomass digestate fraction as a result of pH in the acidic wash step.
Figure 4 illustrates removal of phosphorus from a solid biomass digestate fraction as a result of pH, temperature and time.
Figure 5 illustrates removal of phosphorus from liquid wash fractions.
Figure 6 illustrates the amount of additional methane produced as a result of different post-treatments.

### DETAILED DESCRIPTION

As used herein, the term "non-pressurized" is intended to mean ambient pressure, which at average sea level is a pressure around 1 bar = 101 kPa.

As used herein, the term "pressurized" is intended to mean a pressure significantly higher than ambient pressure. Usually this may be around 10-20 bar, especially in the field of biogas production.

As used herein "biomass digestate" is intended to mean a product of anaerobic digestion (AD) of soft biomass and represents the digested material, which is removed from the AD reactor (digestor) after recovery of biogas. Digestate is normally liquid.

As used herein, the term "soft biomass" is intended to mean non-wood lignocellulosic biomass, comprising cellulose, hemicellulose and lignin. Soft biomass may e.g. be wheat straw, corn stover, rice straw, grass, and bagasse.

Lignocellulosic biomass usually comprises crystalline cellulose fibrils intercalated within a loosely organized matrix of hemicellulose and sealed within an environment rich in hydrophobic lignin. While cellulose itself comprises long, straight chain polymers of D-glucose, hemicellulose is a heterogeneous mixture of short, branched-chain carbohydrates including monomers of all the 5-carbon aldopentoses (C5 sugars) as well as some 6- carbon (C6) sugars including glucose and mannose. Lignin is a highly heterogeneous polymer, lacking any particular primary structure, and comprising hydrophobic phenylpropanoid monomers. Suitable lignocellulosic biomass typically comprises cellulose in amounts between 20 and 50 % of dry mass prior to pretreatment, lignin in amounts between 10 and 40 % of dry mass prior to pretreatment, and hemicellulose in amounts between 15 and 40%.

As used herein, the term "liquid fraction" is intended to mean the fraction having the lowest dry matter after a separation step. The amount of suspended solids is normally around 4% per weight, but typically varies from 0 to less than 10%.

As used herein, the term "solid fraction" intended to mean the fraction having the highest dry matter after a separation step. The amount of suspended solids is normally around 20-25% per weight, but may vary from 10-95%.

As used herein, the term "anaerobic digestion" is intended to mean a collection of processes by which microorganisms break down break down biodegradable material in the absence of oxygen. Anaerobic digestion usually begins with bacterial hydrolysis of the input materials. Insoluble organic polymers, such as carbohydrates, are broken down to soluble derivatives that become available for other bacteria. Acidogenic bacteria may then convert the sugars and amino acids into carbon dioxide, hydrogen, ammonia and organic acids. These bacteria may convert these resulting organic acids into acetic acid, along with additional ammonia, hydrogen, and carbon dioxide. Finally, methanogens may convert these products to methane and carbon dioxide. Anaerobic digestion is widely used as a source of renewable energy. The process produces a biogas, comprising mostly methane and carbon dioxide.

As used herein, the term "pretreatment" is intended to mean a treatment of a biomass prior to a first anaerobic digestion step. This may preferably be a thermal heat treatment equal to or less than 100 degrees Celsius at low pressure of less than 2 bars, advantageously at ambient pressure.

As used herein, the term "posttreatment" is intended to mean a treatment of a biomass subsequent to the acidic wash step and prior to anaerobic digestion of the posttreated solid wash fraction. The posttreatment increases the yield of biogas in the subsequent anaerobic digestion (of the posttreated solid wash fraction). A purpose of the posttreatment is thus to increase the digestability of the solid wash fraction prior to anaerobic digestion.

As used herein nitrogen (N) as is intended to mean the sum of organic bound nitrogen, such as the nitrogen in amines, and inorganic bound nitrogen, such as e.g. NH₃ and NH₄⁺.

As used herein phosphorus/phosphorous (P) is intended to mean the sum of organic bound P and inorganic bound P, such as e.g. PO₄ ions (e.g. PO₄⁻).

As used herein "nutrients" is intended to mean macronutrients for plants, such as nitrogen (N), phosphorus (P), potassium (K), calcium (Ca) and sulfur (S).

As used herein, the singular forms "a", "an" and "the" include plural referents unless the context clearly dictates otherwise.

As used herein, "at least one" is intended to mean one or more, i.e. 1, 2, 3, 4, 5, 6, 7, 8, 9, 10, etc.

As used herein, the term "biogas" is intended to mean methane gas and carbondioxide gas obtained from degradation of biological material, such as biomass.

As used herein, the term "inoculum" is intended to mean organic material containing bacteria, such as digested (degassed) biomass from an existing biogas facility or animal waste.

As used herein, the term "dry matter" is intended to mean the residual when water is evaporated.

As used herein, the term volatile solids (VS) shall mean the organic part of dry matter.

Separation may for example be performed with a screw press, filter press or decanter.

### Abbreviations

VS = volatile solids
5-HMF = 5-(hydroxymethyl)furfural
TS = total solids

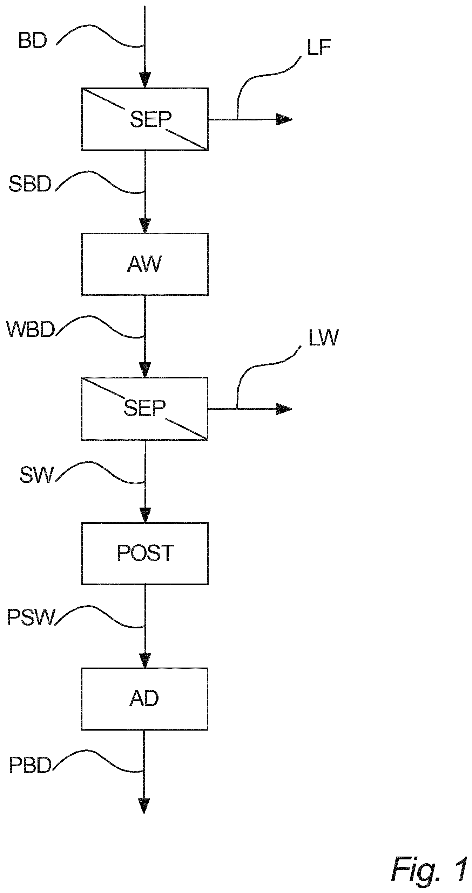

Referring to figure 1, a method for processing biomass digestate according to an embodiment of the invention is disclosed.

Biomass digestate (which may be pretreated) is separated into a solid biomass digestate fraction, such as a fiber fraction, and a liquid fraction. The liquid fraction contains a lower phosphorus concentration compared to the digestate and can be used as fertilizer.

The solid fraction is mixed with acid in a wash reactor in which phosphorus will be dissolved from fibers into the liquid phase. Afterwards the slurry is separated into a solid washed fiber fraction and a liquid wash fraction.

The solid wash fraction (fiber fraction) is posttreated to increase the digestibility of the fiber. The posttreated solid wash fraction is transferred to another biogas reactor and a second anaerobic digestion process is performed to produce more biogas.

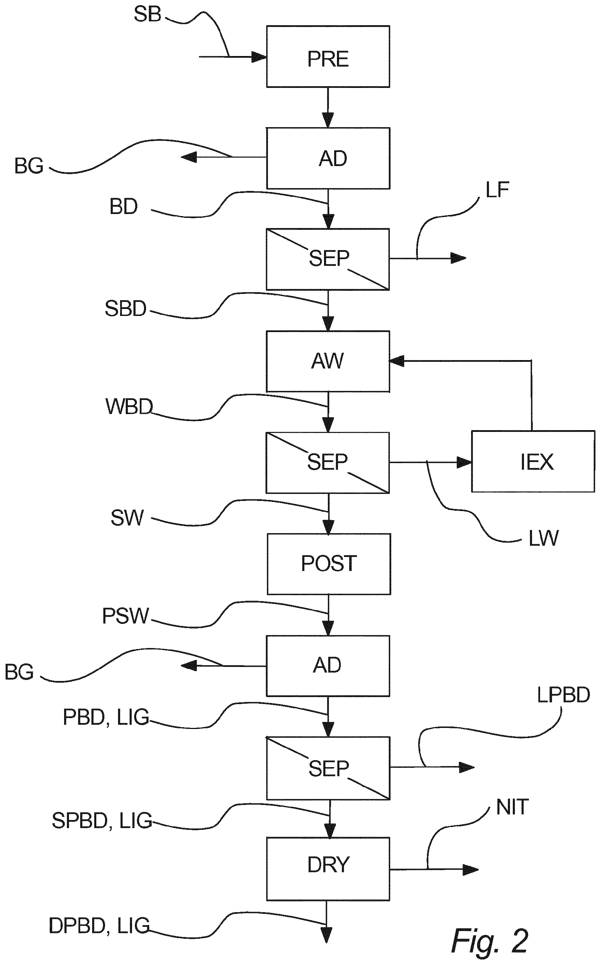

Referring to figure 2, a method for processing biomass digestate according to an embodiment of the invention is disclosed.

Biomass digestate (which may be pretreated) is separated into a solid biomass digestate fraction, such as a fiber fraction, and a liquid fraction. The liquid fraction contains a lower phosphorus concentration compared to the digestate and can be used as fertilizer. The solid fraction is mixed with acid in a wash reactor in which the phosphorus will be dissolved from fibers into the liquid phase. Afterwards the slurry is separated into a solid washed fiber fraction and a liquid wash fraction. The liquid wash fraction may e.g. be transferred to an ion exchange column, in which the phosphorus binds to the resin in the ion exchange column. The ion exchanged liquid wash fraction contains a very low amount of phosphorus and may advantageously be transferred back to the wash reactor.

The phosphorus bound to the resin may be released by e.g. increasing the pH with NaOH, and thereby producing Na₃PO₄. The solid wash fraction (fiber fraction) is post treated to increase the digestibility of the fiber. The posttreated solid wash fraction is transferred to another biogas reactor to produce more biogas. After anaerobic digestion in a biogas process, the slurry may be pressed i.e. separated into a posttreated biomass digestate liquid fraction and a posttreated biomass digestate solid fraction, which may be referred to as a lignin fraction, since this fraction comprises high amounts of lignin. The posttreated biomass digestate liquid fraction may be transferred back to the biogas reactor. The lignin fraction can then be dried. In the dryer, the ammonium will evaporate and the lignin will be dry and of a high purity.

### EXAMPLES

### Example 1: Washing phosphorus out of fiber with sulphuric acid and hydrochloric acid

This example describes how to wash the phosphorus out of biomass digestate, such as a solid biomass digestate fraction, with acid. A fixed amount of fiber i.e. solid biomass digestate has been mixed with different types of acid with varying pH.

Materials and methods:
- Fiber, produced from separating digestate from a biogas plant into a solid biomass digestate fraction
- Water
- Sulphuric acid (H₂SO₄)
- Hydrochloric acid (HCl)

Four washes were made to test sulphuric acid and hydrochloric acid at different pH. 900 g of water was mixed with sulphuric acid and hydrochloric acid, respectively. Two samples were mixed to a pH of 1 and two samples to a pH of 3. 90 g of fiber was added to each of the four samples and mixed. After 1 h at 25 °C, fiber and wash water were separated and phosphorus was measured the liquid wash fraction as well as in an untreated fiber sample, in order to find out how much phosphorus was separated into the liquid wash fraction.

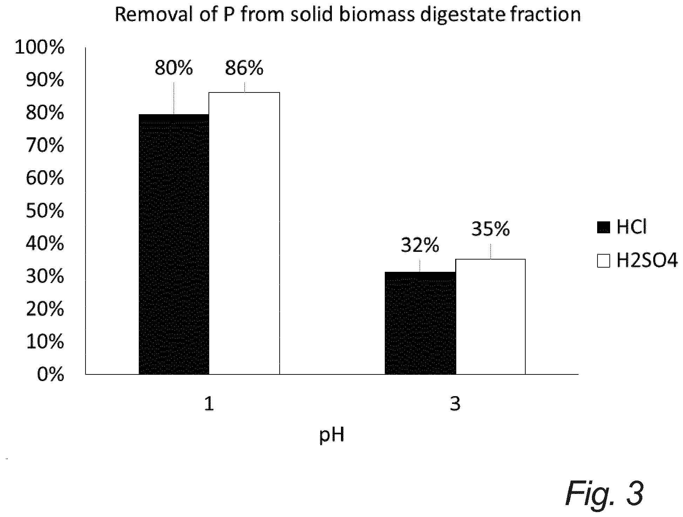

Results:
The results show that pH of the acid has a large impact on the dilution of phosphorus into the wash-liquid. The lower the pH, the more phosphorus is removed from the fiber, as seen on Figure 3.

### Example 2: Washing phosphorus out of the solid biomass digestate fraction with oxalic acid

This example describes how to wash the phosphorus out of solid biomass digestate fraction with oxalic acid. A fixed amount of solid biomass digestate fraction has been mixed with oxalic acid with varying pH, incubation temperature and incubation time.

Materials and methods:
- Solid biomass digestate fraction (fiber fraction, produced from separating digestate from a biogas plant)
- Water
- Oxalic acid (C₂H₂O₄)

A Design of Experiments (DOE) software "MODDE Go" (version 12.1.0.5491, Mar 23 2018) was used to design the experiment and analyze the results. Washes were made with oxalic acid in which three factors were varied; pH of solution, incubation temperature and incubation time. The pH were 0.79, 0.92 and 1.26, the incubation temperatures were 40 °C, 70 °C and 100 °C, and the incubation times were 10 min, 2 hours, and 3 hours and 50 min. For each wash, 900 g of water was mixed with oxalic acid to the desired pH and 90 g of solid biomass digestate fraction was added and mixed. The factors were combined as shown in Table 1.

**Table 1: Scheme of how the factors are combined in the different samples.**

| **Sample** | **pH of acid solution** | **Incubation temperature (°C)** | **Incubation time** |
|---|---|---|---|
| 1 | 1.26 | 40 | 10 min |
| 2 | 1.26 | 100 | 10 min |
| 3 | 1.26 | 70 | 2 hours |
| 4 | 1.26 | 40 | 3 hours 50 min |
| 5 | 1.26 | 100 | 3 hours 50 min |
| 6 | 0.79 | 40 | 10 min |
| 7 | 0.79 | 100 | 10min |
| 8 | 0.79 | 70 | 2 hours |
| 9 | 0.79 | 40 | 3 hours 50 min |
| 10 | 0.79 | 100 | 3 hours 50 min |
| 11 | 0.92 | 40 | 10min |
| 12 | 0.92 | 100 | 10min |
| 13 | 0.92 | 70 | 2 hours |
| 14 | 0.92 | 70 | 2 hours |
| 15 | 0.92 | 70 | 2 hours |
| 16 | 0.92 | 40 | 3 hours 50 min |
| 17 | 0.92 | 100 | 3 hours 50 min |

After each wash, the washed biomass digestate was separated into a solid fraction and a liquid wash fraction and phosphorus was measured on the liquid wash fraction as well as on an untreated solid biomass digestate fraction sample, in order to find out how much phosphorus was diluted (i.e. removed from the solid fraction) in the liquid wash fraction.

### Results:

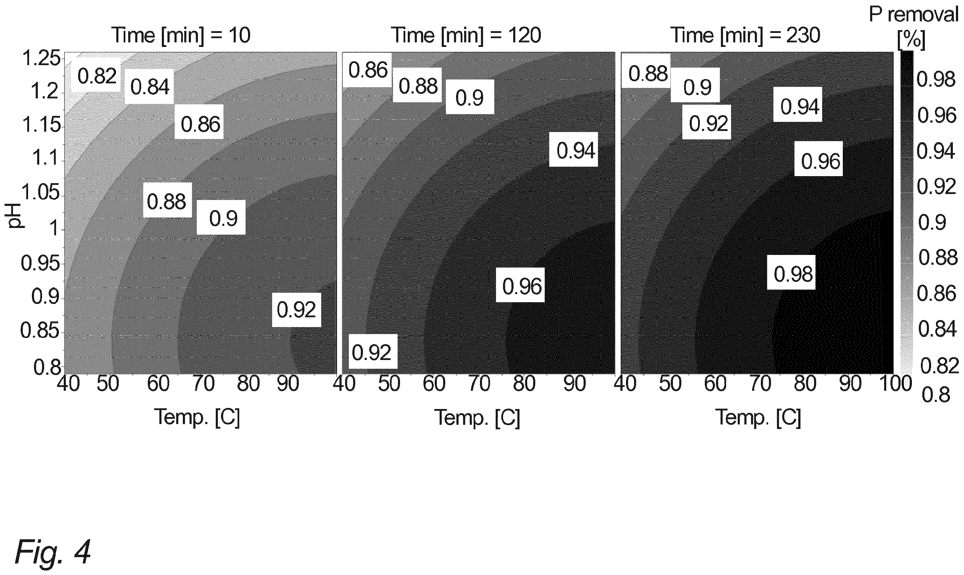

As the experiment was designed with use of a statistical design, a function is calculated on basis of all the data points by the program MODDE Go, and the result of the experiments can thus be seen in Figure 4. Selected raw data points are shown in table 2a-2b, however one should not rely entirely on a single value but instead look at the function calculated on basis of all the data points as shown in Figure 4. Sample 13, 14 and 15 are replicates, and from these the uncertainty of the results is found to be +/-1%. The function has a high R2 value of 88% which is the percent of the variation of the response explained by the model.

The results show that it is possible to remove 99% of the phosphorus from the solid biomass digestate fraction. From figure 4, one can see that the lower the pH is and/or the higher the temperature and/or the longer the washing time, the more phosphorous is removed from the solid biomass digestate fraction (and thus present in the liquid wash fraction).

**Table 2a: Raw data points used to calculate the function illustrated in Figure 4. Amount of phosphorus removed from the solid biomass digestate fraction by washing with an aqueous acidic solution.**

| **Sample** | 1 | 2 | 3 | 4 | 5 | 6 | 7 | 8 | 9 | 10 |
|---|---|---|---|---|---|---|---|---|---|---|
| **P removal** | 75% | 85% | 90% | 89% | 94% | 89% | 95% | 96% | 94% | 96% |

**Table 2b: Raw data points used to calculate the function illustrated in Figure 4. Amount of phosphorus removed from the solid biomass digestate fraction by washing with an aqueous acidic solution.**

| **Sample** | 11 | 12 | 13 | 14 | 15 | 16 | 17 |
|---|---|---|---|---|---|---|---|
| **P removal** | 85% | 91% | 96% | 94% | 95% | 91% | 99% |

### Example 3: Removing phosphorus from the wash-liquid fraction

This example describes how to remove the phosphorus by means of ion exchange from the liquid wash fraction obtained from the acidic wash step.

An acid wash step has been performed prior. The wash was made by mixing 900 g of water with oxalic acid to a pH of 1 and adding 90 g of fiber (solid biomass digestate fraction produced from separating digestate from a biogas plant) with an incubation temperature of 25 °C and an incubation time of 15 hours. Afterwards the washed biomass digestate was separated into a solid wash fraction (fiber fraction) and liquid wash fraction, and the liquid wash fraction was treated.

Materials and methods:
- Liquid wash fraction obtained from acid wash of solid biomass digestate fraction (type of wash is described above)
- Resin 1 (Lewatit FO 36, Lanxess)
- Resin 2 (Amberlite MB 3, Merck)

20 g of liquid wash fraction was mixed with 20 g of resin 1, and 20 g of liquid wash fraction was mixed with 20 g of resin 2 in blue cap bottles. The bottles were incubated at 50 °C for an hour with stirring, and afterwards they were centrifuged to separate the resins from the liquid wash fraction. Phosphorus was measured in the liquid wash fraction without subjecting it to ion exchange, and the two ion exchange treated liquid wash fractions to find out how much phosphorus the two resins are able to remove.

### Results:

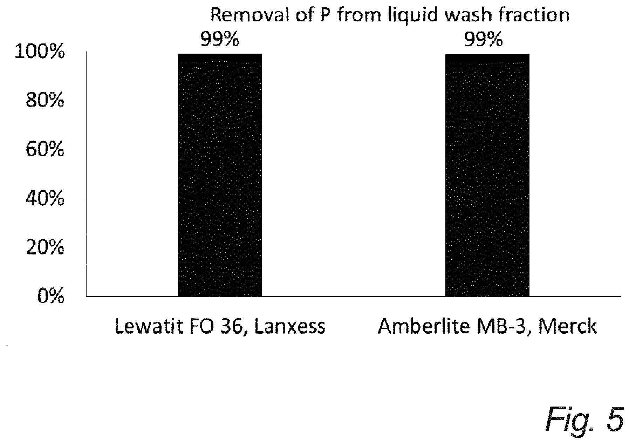

By using ion exchange, it is possible to remove 99% of the phosphorus in the liquid wash fraction obtained from acid wash of solid biomass digestate fraction, as seen in Figure 5. Furthermore, both resins tested can be used for phosphorus removal.

### Example 4: Extra methane as a result of post-treatment

This example describes how to produce extra biogas from biomass digestate as a result of different types of post-treatment of solid wash fractions.

Prior to the post-treatments, an acidic wash of solid biomass digestate fractions was performed. The acidic wash was made by mixing 891 g of water with 9 g of oxalic acid so it had a concentration of 0.1 mol/L and adding 90 g of solid biomass digestate (produced from separating digestate from a biogas plant) with an incubation temperature of 50 °C and an incubation time of 3 hours. Afterwards the washed biomass digestate was separated into a solid wash fraction and a liquid wash fraction, and the solid wash fraction was post-treated.

Materials and methods:
- Solid wash fraction from acid wash (type of wash is described above)
- Water
- Citric acid
- Sodium hydroxide (NaOH)
- Laccase
- Inoculum (reactor liquid from a biogas plant)

Four different samples for posttreatment and subsequent anaerobic digestion were prepared. The first sample was just solid wash fraction from acid wash with no post-treatment. The second sample was solid wash fraction from acid wash, which was post-treated with steam explosion. The third sample was solid wash fraction from acid wash, which was post-treated with laccase. The fourth sample was solid wash fraction from acid wash, which was post-treated with steam explosion and afterwards laccase. The steam explosion was performed by mixing 40 g of solid wash fraction and 160 g of water in a Parr Reactor, which was run for 15 min at 180 °C.

The laccase treatment was performed by mixing 8 g of solid wash fraction with 90 g of a 0.1 M citric acid buffer adjusted to pH 5 with NaOH. 0.6 g laccase/g TS solid wash fraction was added and the sample was stirred for 24 hours at 250 rpm in a shaking incubator at 45 °C.

After each post-treatment, the four samples were adjusted to a pH of 8 with NaOH and mixed with inoculum and anaerobic digested for 15 days at a temperature of 52 °C.

### Results:

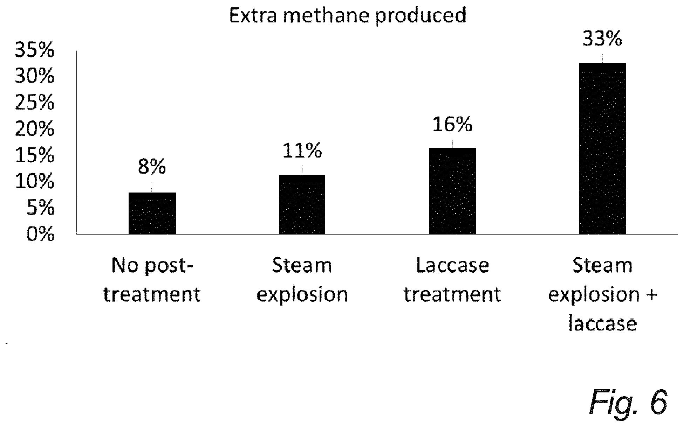

It is possible to produce up to 33% extra methane by post-treating the solid wash fraction with steam explosion followed by laccase treatment, as seen in Figure 6.

### Example 5: Lignin product description

The posttreated biomass digestate from Example 4 were dried and the resulting dried posttreated biomass digestate was a lignin fraction comprising the following:
Lignin: 36 %
Water content: 12%
Ash: 4.5%
Chlor: 0.013%
Sulphur: 0.4%
Calorific value: 18. MJ/kg

### Example 6.

Biomass digestate may for example advantageously be provided by subjecting a soft biomass to heating in a non-pressurized pretreatment step at a pretreatment temperature between 65 to 100 degrees Celsius at ambient pressure. The heating may for example be performed in a tank or container with or without stirring. The retention time in the pretreatment step is usually short, such as less than one hour or less than 30 minutes, but it may also be longer if this may be convenient. The pretreated biomass may then be conveyed into a further tank to be anaerobic digested to provide a biomass digestate. The anaerobic digestion may be performed for example by the addition of bacteria. The anaerobic digestion is usually performed at a pH around neutral, such as for example between pH 6-8. An important degradation product, which may be obtained from the anaerobic digestion step is biogas, comprising methane and/or carbon dioxide.

### FIGURE REFERENCES

DRY. Drying step
BD. Biomass digestate
SEP. Separation step
LF. Liquid fraction
SBD. Solid biomass digestate fraction
AW. Acidic wash step
WBD. Washed biomass digestate,
LW. Liquid wash fraction
SW. Solid wash fraction
POST. Posttreating
PSW. Posttreated solid wash fraction
PRE. pretreating
AD. Anaerobic digesting
PBD. Posttreated biomass digestate
IEX. ion exchange
LPBD. Liquid posttreated biomass digestate fraction
SPBD. Solid posttreated biomass digestate fraction
DPBD. Dried posttreated biomass digestate
LIG. Lignin
DRY. Drying step
BG. Biogas
SB. Soft Biomass
NIT. Nitrogen

## Claims

1. A method for processing biomass digestate, the method comprising the steps of
- providing a biomass digestate (BD),
- subjecting the biomass digestate to a separation step (SEP) into a liquid fraction (LF) and a solid biomass digestate fraction (SBD),
- subjecting the solid biomass digestate fraction (SBD) to an acidic wash step (AW) at a pressure below 2 bar to obtain a washed biomass digestate (WBD),
- separating (SEP) the washed biomass digestate (WBD) into a liquid wash fraction (LW) and a solid wash fraction (SW),
- posttreating (POST) the solid wash fraction (SW) to obtain a posttreated solid wash fraction (PSW) and
- anaerobic digesting (AD) the posttreated solid wash fraction (PSW) to obtain a posttreated biomass digestate (PBD)
wherein the amount of phosphorous in the solid wash fraction (SW) is reduced compared to amount of phosphorous in the solid biomass digestate fraction (SBD).

2. The method according to claim 1, wherein the acidic wash step (AW) is non-pressurized.

3. The method according to claim 1 or 2, wherein the amount of phosphorous in the solid wash fraction (SW) is less than 10% by weight of the phosphorous in the biomass digestate (BD).

4. The method according to any of the preceding claims, wherein the pH in the acidic wash step (AW) is lower than 6, such as between 0 to 6, or lower than 4, such as between 0 to 4.

5. The method according to any of the preceding claims, wherein the temperature in the acidic wash step (AW) is between 50 to 100 degrees Celsius.

6. The method according to any of the preceding claims, wherein the acidic wash step (AW) is performed for between 10 minutes to 24 hours, such as between 10 minutes to 10 hours, such as between 10 minutes and 5 hours.

7. The method according to any of the preceding claims, wherein the concentration of the acid in the acidic wash step (AW) is between 0.01 to 1 mol/liter, such as between 0.04 to 0.5 mol/liter, such as between 0.04 to 0.2 mol/liter.

8. The method according to any of the preceding claims, wherein the acid in the acidic wash step (AW) is a strong acid.

9. The method according to any of the preceding claims, wherein the acid in the acidic wash step (AW) is a weak acid.

10. The method according to any of the preceding claims, wherein the amount of phosphorus in the liquid wash fraction (LW) is reduced to obtain a low phosphorus liquid fraction.

11. The method according to any of the preceding claims, wherein the amount of phosphorus in the liquid wash fraction (LW) is reduced by subjecting the liquid wash fraction (LW) to ion exchange (IEX).

12. The method according to any of the preceding claims, wherein the ion exchange (IEX) is performed by passing the liquid wash fraction (LW) over a resin.

13. The method according to any of the preceding claims, wherein the liquid wash fraction (LW) is recirculated into the acidic wash step (AW).

14. The method according to any of the preceding claims, wherein the biomass digestate (BD) is provided by heating a soft biomass (SB) in a pretreatment step (PRE) followed by anaerobic digesting (AD) of the soft biomass (SB) to obtain the biomass digestate (BD).

15. The method according to any of the preceding claims, wherein the pretreatment (PRE) is non-pressurized.

16. The method according to any of the preceding claims, wherein the pretreatment (PRE) temperature is between 65 to 100 degrees Celsius, such as between 65 to 90 degrees Celsius, such as between 65 to 80 degrees Celsius or such as between 70 to 90 degrees Celsius.

17. The method according to any of the preceding claims, wherein the pretreatment step (PRE) is performed for 2 hours or less.

18. The method according to any of the preceding claims, wherein the pH in the pretreatment step (PRE) is between 2 to 9, such as between 3 to 7, such as between 3 to 4.

19. The method according to any of the preceding claims, wherein acid and/or food waste is added to the soft biomass (SB).

20. The method according to any of the preceding claims, wherein the anaerobic digesting (AD) of the pretreated biomass is performed for less than 10 days.

21. The method according to any of the preceding claims, wherein the amount of hemicellulose in the biomass digestate after the first anaerobic digesting step is less than 40% by weight of the amount of hemicellulose in the soft biomass (SB) prior to the first anaerobic digesting step, such as less than 30% by weight.

22. The method according to any of the preceding claims, wherein the posttreatment (POST) step is pressurized.

23. The method according to any of the preceding claims, wherein the posttreatment (POST) temperature is between 150 to 230 degrees Celsius, such as between 170 to 210 degrees Celsius, such as between 180 to 200 degrees Celsius.

24. The method according to any of the preceding claims, wherein the posttreatment (POST) step is performed for less than 1 hour.

25. The method according to any of the preceding claims, wherein the posttreatment (POST) is performed by steam explosion and/or laccase treatment.

26. The method according to any of the preceding claims, wherein the posttreatment (POST) is performed by bacteria or fungi treatment.

27. The method according to any of the preceding claims, wherein furfural and 5-HMF and 2-furioc acid are generated in a combined amount of less than 5% by weight in the post treatment (POST) step.

28. The method according to any of the preceding claims, wherein anaerobic digesting (AD) of the posttreated solid wash fraction (PSW) is performed for less than 30 days.

29. The method according to any of the preceding claims, wherein the method further comprises a further separation step (SEP) after anaerobic digesting (AD) the posttreated solid wash fraction (PSW) to obtain a liquid posttreated biomass digestate fraction (LPBD) and a solid posttreated biomass digestate fraction (SPBD).

30. The method according to any of the preceding claims, wherein the method further comprises a drying step (DRY) of the solid posttreated biomass digestate fraction (SPBD) to obtain a dried posttreated biomass digestate (DPBD).

31. The method according to any of the preceding claims, wherein the amount of nitrogen in the dried posttreated biomass digestate (DPBD) is reduced by at least 50%, such as at least 60%, such as at least 70% or such as at least 80% by weight of the nitrogen comprised in the biomass digestate (BD) entering the process.

32. The method according to any of the preceding claims, wherein lignin (LIG) is obtained from the method in a purity of at least 30% by weight, such as at least 35% by weight, such as at least 40% by weight.

33. The method according to any of the preceding claims, wherein lignin (LIG) is comprised in the posttreated biomass digestate (PBD) or dried posttreated biomass digestate (DPBD) in an amount of more than 30% by weight, such as in an amount of more than 35% by weight, such as in an amount of more than 40% by weight.

34. The method according to any of the preceding claims, wherein biogas (BG) is obtained from at least the anaerobic digesting step (AD) of the posttreated solid wash fraction (PSW).

35. The method according to any of the preceding claims, wherein the method further comprises recirculation of at least a part of a liquid fraction from any separation step (SEP) to any anaerobic digesting step (AD) and/or the pretreatment step (PRE).
